# EUROPÄISCHE PATENTANMELDUNG

(11) **EP 2 158 866 A1**
(43) Veröffentlichungstag der Anmeldung: **03.03.2010**
(21) Anmeldenummer: 09010952.1
(22) Anmeldetag: 27.08.2009
(51) Int. Cl.: A61B 18/02, A61B 18/14, A61B 17/22

(54) **Vorrichtung zum Ablatieren der Mündung der Lungenvenen**

(30) Priorität: 29.08.2008 DE 102008045038
(71) Anmelder: Osypka, Peter, 79618 Rheinfelden-Herten (DE)
(72) Erfinder: Osypka, Peter, 79618 Rheinfelden-Herten (DE)
(74) Vertreter: Maucher, Wolfgang

(57) **Zusammenfassung**

Eine Vorrichtung (1) dient zum Ablatieren oder Koagulieren der Mündung (2) oder des Ausgangs von Lungenvenen (3) im linken Vorhof (4) eines menschlichen Herzens, wodurch ein Vorhofflimmern oder die Neigung dazu beseitigt werden kann. Die Vorrichtung (1) hat dazu eine Einrichtung (5) zum Zerstören des Gewebes an der Mündung (2) und außerdem wenigstens einen in die zu behandelnde Lungenvene (3) von der Mündung (2) aus einführbaren Verschluss (6), der in Gebrauchsstellung den Zufluss von Blut aus der Lunge in den Vorhof (4) und damit zu der Mündung (2) mit Abstand zu dieser Mündung (2) unterbindet. Durch einen an der Vorrichtung (1) anlegbaren, zwischen Einrichtung (5) und Verschluss (6) wirksamen Saugdruck kann der Querschnitt der Lungenvene (3) im Mündungsbereich verkleinert werden, was sie Effektivität bei der Zerstörung des Gewebes an der Mündung (2) verbessert und/oder eine kleiner bemessene Einrichtung (5) erlaubt (Fig.1).

## Beschreibung

Die Erfindung betrifft eine Vorrichtung zum Ablatieren oder Koagulieren der Mündung oder des Ausgangs der Lungenvenen im linken Vorhof eines menschlichen Herzens zur Beseitigung eines Vorhofflimmerns, mit einer Einrichtung zum Zerstören des Gewebes am Ausgang der Lungenvenen, wobei zu der Vorrichtung wenigstens ein in die zu behandelnde Lungenvene einführbarer Verschluss gehört, der in Gebrauchsstellung den Zufluss von Blut aus der Lunge in den Vorhof mit Abstand zur Mündung oder zu dem Ausgang der Lungenvene unterbindet.

Eine vergleichbare Vorrichtung ist aus der US 2007/0078451 A1 und aus der US 6 814 733 B2 bekannt.

Mit derartigen Vorrichtungen kann das Gewebe im Mündungsbereich einer Lungenvene durch Koagulation zerstört werden. Der Verschluss ermöglicht es dabei, die zu behandelnde Stelle eventuell erwärmendes Blut von der zu behandelnden Mündung der Lungenvene fernzuhalten und somit einen Behandlungsvorgang zum Beispiel mittels Kälte nicht zu verzögern und auch eine Ablation mittels Hochfrequenz nicht zu stören. Auch entfällt die Notwendigkeit für den Operateur, die Behandlungsvorrichtung gegen den Blutstrom und gegen den Blutdruck andrücken und festhalten zu müssen. Eine gewisse Menge an Blut verbleibt jedoch zwischen dem Verschluss und der Behandlungsstelle und kann somit immer noch eine Verzögerung bewirken.

Es besteht deshalb die Aufgabe, eine Vorrichtung der eingangs genannten Art zu schaffen, mit welcher das Gewebe am Ausgang der Lungenvene in möglichst kurzer Zeit zerstört werden kann, wobei die Einrichtung zum Zerstören des Gewebes eine möglichst geringe Abmessung haben können soll.

Zur Lösung dieser Aufgabe ist vorgesehen, dass die Vorrichtung eine Saugeinrichtung aufweist, deren Einlass in Gebrauchsstellung zwischen dem Verschluss und der Einrichtung zur Gewebezerstörung angeordnet ist. Unabhängig davon, ob die Einrichtung zur Gewebezerstörung eine Koagulationselektrode oder ein Behältnis für Flüssiggas ist, kann auf diese Weise ein Rest von Blut aus dem der Mündung nahen Venenbereich vor dem eigentlichen Behandlungsbeginn abgesaugt werden, so dass die Behandlung selbst noch besser und in kürzerer Zeit ablaufen kann. Darüber hinaus kann durch eine Saugwirkung auf diesem Venenbereich deren Querschnitt und insbesondere ihr Mündungsbereich verkleinert werden, so dass auch die Einrichtung zum Zerstören des Gewebes nur eine geringere Abmessung benötigt und dennoch das im Mündungsbereich befindliche Gewebe mit großer Sicherheit in relativ kurzer Zeit vollständig zerstören kann.

Der Eintritt in die Saugeinrichtung kann eine gelochte oder durchlöcherte Schlauch- oder Röhrchen- oder Metallhülse sein, die ihrerseits einen schlauch- oder röhrchenförmigen Anschluss zu einer Unterdruckquelle hat. Eine solche gelochte oder gelöcherte oder siebartige Hülse kann die Blutflüssigkeit gut absaugen und hat dennoch eine ausreichende Festigkeit und Stabilität.

Zweckmäßig ist es, wenn der Verschluss als Ballon ausgebildet ist, der durch einen Zuführkatheter vom Vorhof des Herzens in die Lungenvene einführbar und dann aufweitbar ist. Derartige aufweitbare Ballons sind in der Medizin bei der Behandlung von Gefäßen bereits bekannt und haben sich bewährt, so dass die Verwendung eines Ballons als erfindungsgemäßer Verschluss als besonders einfach zu realisierende Ausführungsform angesehen werden kann. Ein derartiger als Ballon ausgebildeter Verschluss erleichtert es, diesen beispielsweise mittels eines Katheters zunächst in den Vorhof des Herzens und von dort in die jeweilige Lungenvene einzuführen, um ihn dann für die Gebrauchsstellung aufzuweiten.

Der Verschluss kann an seiner Peripherie oder Außenseite Verankerungsmittel haben. Dadurch wird die Sicherheit erhöht, dass der Verschluss in seiner Gebrauchslage verbleibt, obwohl das Blut von der Vene her gegen diesen Verschluss drückt.

Als Verankerungsmittel kann an dem Verschluss oder Ballon ein diesen Verschluss oder Ballon umschließendes Geflecht aus Fäden oder Drähten, beispielsweise aus Nitinoldrähten vorgesehen sein, die sich ebenfalls in der Herz- und Gefäßchirurgie bewährt haben.

Dabei kann diese Saughülse auf einer Zuführstange oder auf einem Zuführschaft für den Verschluss koaxial zu dieser Zuführstange, aber mit radialem Abstand dazu angeordnet sein. Somit kann diese Zuführstange für den Verschluss eine Zusatzfunktion erhalten, indem sie auch die Saughülse positioniert und hält.

Die Einrichtung zur Gewebezerstörung im Mündungsbereich der Lungenvenen kann ein mit einem Kältemittel zu füllender Ballon und/oder eine metallische Elektrode sein, die einen ununterbrochenen Umfang für Beaufschlagung der Mündung der jeweiligen Lungenvene hat. Somit braucht weder bei einer Kältebehandlung noch bei einer Koagulation das Instrument oder die Einrichtung zur eigentlichen Gewebezerstörung mehrmals nacheinander an derselben Venenmündung angesetzt zu werden, was vor allem dann begünstigt ist, wenn die Vene im Mündungsbereich durch Unterdruck in ihrem Querschnitt verkleinert ist.

Somit ist es besonders günstig, wenn die gesamte Vorrichtung und die Einrichtung zum Zerstören des Gewebes mit der vorerwähnten Saugeinrichtung kombiniert ist.

Für eine schnellstmögliche Einführung und Einfügung des erfindungsgemäßen Verschlusses in eine zu behandelnde Lungenvene ist es vorteilhaft, wenn die Vorrichtung einen Führungsdraht aufweist und zumindest der Verschluss einen den Führungsdraht verschiebbar umfassenden Kanal oder Durchtritt hat. Somit kann zunächst der Führungsdraht in die jeweilige Lungenvene eingeführt und danach der Verschluss und auch die Einrichtung zum Zerstören des Gewebes am Ausgang der Lungenvene über diesen Führungskanal in ihre Gebrauchsstellung gebracht werden. Dabei kann die Einführtiefe des Verschlusses aufgrund von dessen Abstand von der Einrichtung zum Zerstören des Gewebes vorgegeben sein.

Noch einfacher ist die Einführung des Verschlusses und der Einrichtung zum Zerstören des Gewebes, wenn nach dem Installieren des Führungsdrahtes zunächst ein Zuführkatheter zusammen mit dem noch darin befindlichen Verschluss und der Einrichtung zum Zerstören des Gewebes in die Lungevene eingeführt und danach der Katheter zurückgezogen wird, woraufhin sich der Verschluss selbsttätig oder durch einen Aufweit-Vorgang von außen her seine Gebrauchsstellung erhält.

Der Verschluss und die Einrichtung zum Zerstören des Gewebes kann mittels einer in Gebrauchsstellung gespannten Zugfeder verbunden sein. Somit kann zunächst der Verschluss in seine Gebrauchslage gebracht werden, während die Einrichtung zum Zerstören des Gewebes noch von dem Zuführkatheter umschlossen ist, und dann kann mit dem Zurückziehen des Katheters auch diese Einrichtung unter Spannung der Zugfeder zurückgezogen und aufgeweitet werden, so dass sie dann durch die Kraft der Zugfeder in Gebrauchsstellung an der Mündung der Lungenvene anliegt und angedrückt ist und eine entsprechend intensive Wirkung auf das Gewebe ausüben kann. Die Zugfeder kann dabei aufgrund ihrer vor allem in Gebrauchsstellung auseinander gezogenen Gewindegänge eine Absaugung durch diese Gewindegänge hindurch in die entsprechende Saughülse ermöglichen.

Eine besonders zweckmäßige Ausgestaltung der Erfindung kann vorsehen, dass die insbesondere als Zugfeder ausgebildete Verbindung zwischen dem Verschluss und der Einrichtung zum Behandeln oder Koagulieren des Gewebes aus elektrisch leitfähigem Werkstoff oder aus zumindest teilweise blankem Metall besteht und als Elektrode wirkend mit einer Signalaufnahmeeinrichtung verbunden ist. Dadurch ist es möglich, in der Lungenvene nahe ihrer Mündung in die Herzvorkammer dort auftretende Signale mit Hilfe der Verbindung oder Zugfeder zu detektieren um festzustellen, ob an der Mündung genügend Gewebe behandelt oder koaguliert oder zerstört ist, um derartige Signale zu unterbinden. Werden Signale festgestellt, muss die Behandlung fortgesetzt werden. Somit erhält diese Verbindung oder Zugfeder eine Zusatzfunktion als Sensing-Elektrode.

Nachstehend sind Ausführungsbeispiele der Erfindung anhand der Zeichnung näher beschrieben. Es zeigt in zum Teil schematisierter Darstellung:
- Fig. 1: einen Längsschnitt einer erfindungsgemäßen Vor- richtung in Gebrauchsstellung, in welcher der ballonartige, außenseitig Verankerungsmittel aufweisende Verschluss mit Abstand hinter der Mündung einer Lungenvene angeordnet ist, während an der Mündung selbst eine Einrichtung zum Zer- stören des Gewebes in Form eines Behältnisses zur Aufnahme von Flüssiggas und/oder in Form ei- ner an diesem Behältnis umlaufenden Elektrode für Hochfrequenzwärme anliegt, wobei diese Ein- richtung und der Verschluss über eine in Gebrauchsstellung gedehnte Zugfeder verbunden sind,
- Fig. 2: eine der Figur 1 entsprechende Darstellung eines abgewandelten Ausführungsbeispiels, bei welchem der Verschluss und die Zugfeder mit der Anord- nung gemäß Figur 1 übereinstimmen, aber die Ein- richtung zum Zerstören des Gewebes im Bereich der Mündung der Lungenvene als Metallkäfig ins- besondere aus Nitinoldrähten mit Elektroden zur Hochfrequenzablation ausgebildet ist, wobei auch Temperaturfühler vorhanden sein können und der Metallkäfig eine äußere Abdichtung hat,
- Fig. 3: die Lungenvene vor dem Einfügen der Vorrichtung, nachdem ein von dem linken Vorhof des Herzens herkommender Führungsdraht verlegt ist,
- Fig. 4: einen Querschnitt der Vene, in deren Zentrum der Führungsdraht verläuft,
- Fig. 5: einen Längsschnitt der Vene, nachdem ein Schutz- oder Zuführkatheter mit der in ihm befindlichen erfindungsgemäßen Vorrichtung über den Führungs- draht in die Gebrauchslage verschoben ist, aber die Vorrichtung noch in diesem Katheter zusam- mengehalten wird,
- Fig. 6: einen Querschnitt der Vene und des Zuführkathe- ters mit der darin befindlichen Vorrichtung,
- Fig. 7: einen Längsschnitt der Vene mit der Vorrichtung und der an der Mündung befindlichen Einrichtung zum Zerstören des Gewebes, nachdem der Zuführka- theter entfernt und dabei auch die Zugfeder ge- spannt wurde,
- Fig. 8: einen Querschnitt der Vene im Bereich der Zugfe- der,
- Fig. 9: einen Querschnitt der Vene im Bereich der Zugfe- der gemäß Figur 1, nachdem der Innenquerschnitt der Vene zwischen Verschluss und Einrichtung durch Unterdruck verkleinert wurde, sowie
- Fig. 10: einen Schnitt durch ein menschliches Herz mit Blick auf die vier Mündungen der Lungenvenen in dem linken Vorhof mit einem Zuführkatheter für die erfindungsgemäße Vorrichtung.

Bei der nachfolgenden Beschreibung der Ausführungsbeispiele erhalten hinsichtlich ihrer Funktion übereinstimmende Teile auch bei unterschiedlicher Gestaltung oder Formgebung übereinstimmende Bezugszahlen.

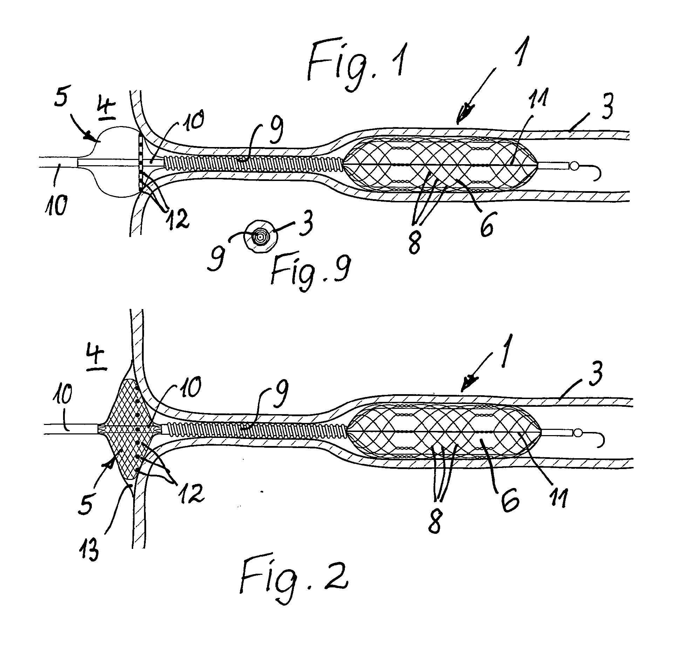

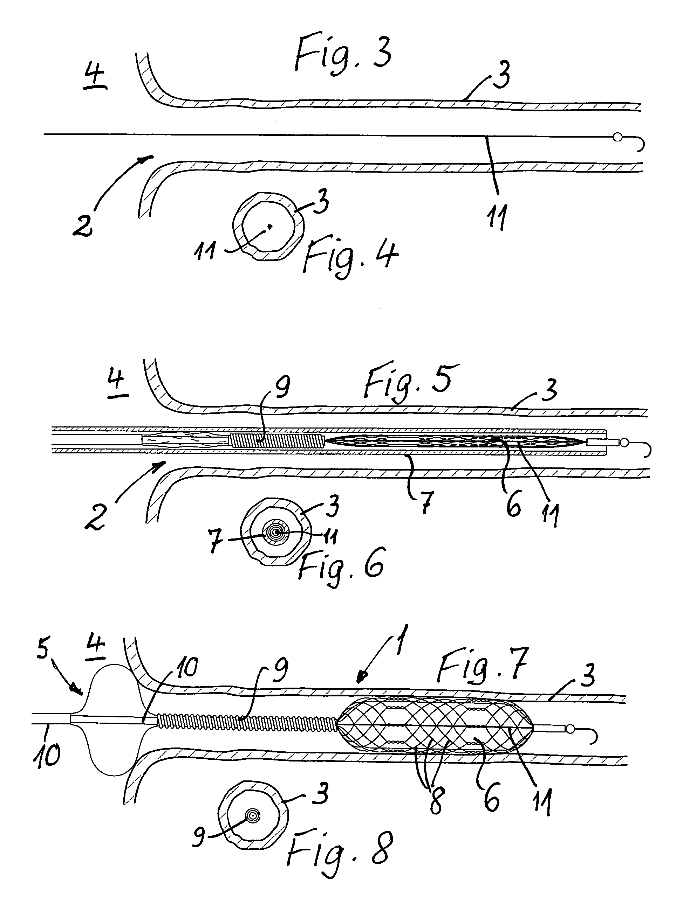

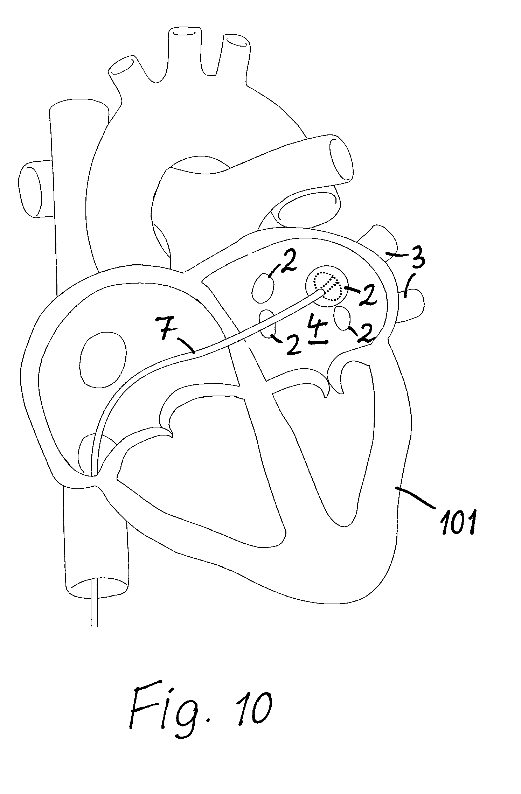

Eine im Ganzen mit 1 bezeichnete, in den Figuren 1 und 2 in abgewandelten Ausführungsbeispielen in Gebrauchsstellung dargestellte Vorrichtung dient zum Ablatieren oder Koagulieren, also zum Zerstören von Gewebe an der Mündung 2 (vgl. vor allem Figur 3, 5 und 10) oder dem Ausgang einer Lungenvene 3 im linken Vorhof 4 eines menschlichen Herzens 101. Durch eine derartige Behandlung kann das Vorhofflimmern oder eine Neigung dazu beseitigt werden. Demgemäß gehört zu der Vorrichtung 1 eine im Ganzen mit 5 bezeichnete Einrichtung zum Zerstören des Gewebes an der Mündung 2, die unterschiedlich ausgebildet sein kann.

In den Figuren 1, 2, 5 und 7 erkennt man, dass zu der Vorrichtung 1 ein in die zu behandelnde Lungenvene 3 einführbarer Verschluss 6 gehört, der in der Gebrauchsstellung gemäß den Figuren 1 und 2 sowie 7 den Zufluss von Blut aus der Lunge in den Vorhof 4 mit Abstand zur Mündung 2 der Lungenvene 3 unterbindet, so dass die Einrichtung 5 bei ihrer Benutzung nicht einem Blutstrom oder dem Blutdruck ausgesetzt ist.

Beim Vergleich der Figuren 1 und 2 mit Figur 5 wird deutlich, dass der Verschluss 6 aus einer Ausgangslage (Figur 5) mit geringer Querschnittsabmessung in die Gebrauchsstellung aufweitbar ist, in welcher er den Querschnitt der Lungenvene 3 ausfüllt und abschließt.

Dabei ist der Verschluss 6 in beiden Ausführungsbeispielen als Ballon ausgebildet, der durch einen Zuführkatheter 7 (vgl. Figur 5 und 6) vom Vorhof 4 des Herzens durch die Mündung 2 in die Lungenvene 3 einführbar und dann nach Rückzug des Zuführkatheters 7 aufweitbar ist.

Gemäß den Figuren 1, 2 und 7 hat der Verschluss 6 an seiner Peripherie oder Außenseite Verankerungsmittel, die im Ausführungsbeispiel als ein den Verschluss 6 oder Ballon außenseitig umschließendes Geflecht 8 aus Fäden oder Drähten, beispielsweise aus Nitinoldrähten, ausgebildet sind. Dabei erkennt man in den Figuren, dass dieses Geflecht 8 mehrere Abschnitte hat, um an die entsprechenden Umfangsbereiche des Ballons angepasst werden zu können.

In den Figuren 1, 2, 5 und 7 erkennt man, dass bei der Vorrichtung 1 der Verschluss 6 und die in den verschiedenen Ausführungsbeispielen unterschiedlich gestaltete Einrichtung 5 zum Zerstören des Gewebes an der Mündung 2 mittels einer in Gebrauchsstellung gespannten Zugfeder 9 verbunden sind. Während diese Zugfeder 9 in Figur 5 noch in ihrer entspannten zusammengezogenen Lage dargestellt ist, weil der Verschluss noch nicht installiert und verankert ist, ist sie in den übrigen Figuren auseinander gezogen und gespannt und bewirkt dadurch, dass die Einrichtung 5 gegen die Mündung 2 gezogen und gehalten wird und ihre Wirkung entsprechend intensiv entfalten kann.

Die im Ausführungsbeispiel als Zugfeder 9 ausgebildete Verbindung zwischen dem Verschluss 6 und der Einrichtung 5 zum Behandeln oder Zerstören von Gewebe besteht aus elektrisch leitfähigem Werkstoff oder blankem Metall und kann in nicht näher dargestellter Weise mit einer Signalaufnahmeeinrichtung verbunden sein, um gleichzeitig auch als Elektrode wirken zu können. Die Zugfeder 9 kann bei einer derartigen Gestaltung in der Lungenvene 3 nahe der Mündung 2 in die Herzvorkammer 4 auftretende Signale detektieren. Treten derartige Signale auf, ist noch nicht genügend Gewebe zum Unterbinden solcher Signale zerstört, d.h. die Behandlung muss fortgesetzt werden. Treten keine Signale mehr auf, kann die Behandlung als abgeschlossen angesehen werden. Somit hat die Zugfeder 9 bei der erwähnten Verbindung mit einer Signalaufnahmeeinrichtung eine Zusatzfunktion als Sensing-Elektrode.

Die Vorrichtung 1 weist außerdem eine nicht näher dargestellte Saugeinrichtung auf, deren Einlass in Gebrauchsstellung zwischen dem Verschluss 6 und der Einrichtung 5 zur Gewerbezerstörung angeordnet ist und sich in den Ausführungsbeispielen innerhalb der Zugfeder 9 befinden kann. Der Eintritt in diese Saugeinrichtung kann eine gelochte oder durchlöcherte Schlauch- oder Röhrchen- oder Metallhülse sein, die einen ebenfalls zu der Saugeinrichtung gehörenden schlauch- oder röhrchenförmigen Anschluss 10 hat, der in den Figuren 1, 2 und 7 innerhalb der Einrichtung 5 und an deren der Mündung 2 abgewandten Bereich angedeutet ist. Dieser Anschluss 10 kann mit einer Saugquelle oder einer Unterdruckeinrichtung verbunden sein, um die erwähnte Saugwirkung ausüben zu können, mit welcher zwischen dem Verschluss 6 und der Einrichtung 5 befindliches Blut aus der Lungenvene 3 abgesaugt werden kann. Durch den Unterdruck wird die Lungenvene 3 in diesem Bereich außerdem in radialer Richtung zusammengezogen oder eingeschnürt, was durch den Vergleich der Figur 7 mit den Figuren 1 und 2 deutlich wird, denn in der endgültigen Gebrauchsstellung gemäß den Figuren 1 und 2 ist an den Anschluss 10 der erwähnte Unter- oder Saugdruck bereits angelegt. Man erkennt bei dem Vergleich der genannten Figuren, dass dadurch auch die Mündung 2 zweckmäßiger Weise verkleinert wird, so dass auch eine entsprechend kleiner bemessene Einrichtung 5 zur Anwendung kommen kann, die dennoch eine intensive Wirkung auf das Gewebe der Mündung 2 ausüben kann, weil sie entsprechend fest daran anliegt und nicht einen von der Lungenvene 3 kommenden Blutdruck oder Blutstrom berücksichtigen muss. Somit entfällt auch die Notwendigkeit, dass der Benutzer der Vorrichtung 1 die Einrichtung 5 während der gesamten Behandlungsdauer ständig an die zu behandelnde Stelle andrückt.

Durch die unter der Saugwirkung im Querschnitt verkleinerte Lungenvene 3 wird der Kontakt mit der als Sensing-Elektrode ausgebildeten Zugfeder 9 begünstigt oder verbessert.

In den Figuren ist außerdem zu sehen, dass die Vorrichtung 1 einen Führungsdraht 11 aufweist und zumindest der Verschluss 6 einen den Führungsdraht 11 verschiebbar umfassenden Kanal oder Durchtritt hat, der in Längsrichtung verläuft. Somit kann zunächst gemäß Figur 3 und 4 dieser Führungsdraht 11 vom linken Vorhof 4 des Herzens über die Mündung 2 in die Lungen- oder pulmonale Vene eingeführt werden. Danach kann der Zuführkatheter 7 gemäß Figur 5, 6 und 10 über diesen Führungsdraht in die Gebrauchslage verschoben werden, wobei er gleichzeitig die zunächst in dem Zuführkatheter 7 befindliche Vorrichtung 1 in die in Figur 5 erkennbare Position bringt.

Danach kann der Zuführkatheter 7 allmählich zurückgezogen werden, so dass er zunächst den Verschluss 6 freigibt, der dann schon aufgeweitet werden kann. Bei der weiteren Zurückziehung des Katheters 7 wird dann schließlich auch die Einrichtung 5 freigegeben, die dabei soweit vor die Mündung verstellt werden kann, dass sie durch die dabei gespannte Zugfeder 9 in aufgeweiteter Lage an die Mündung 2 angelegt und angedrückt wird.

Diese Einrichtung 5 zur Gewebezerstörung kann gemäß Figur 1 und 7 ein mit einem Kältemittel zu füllender Ballon und gemäß Figur 2 eine metallische Elektrode beispielsweise bestehend aus einem Metallkäfig mit darauf angeordneten einzelnen Elektroden 12 sein.

In Figur 1 ist angedeutet, dass auch ein solcher aus einzelnen Elektroden 12 bestehender Ring auf dem Ballon angeordnet sein kann, der mit einem Kältemittel, z.B. flüssigem Gas, gefüllt werde kann, so dass die Ausführungsform gemäß Figur 1 wahlweise mit einem Kältemittel oder mit Elektroden oder gegebenenfalls mit beiden benutzt werden könnte.

Somit kann an der Mündung 2 das Gewebe entweder über einen mit einem Kältemittel gefüllten Ballon oder mit Hilfe von Elektroden 12 zerstört werden, wobei aufgrund der beschriebenen Anordnung und insbesondere auch der Möglichkeit, den mündungsnahen Teil der Lungenvene 3 und auch die Mündung 2 selbst durch den Unterdruck in den Außenabmessungen zu verringern, auch kleinere Einrichtungen 5 schon zu einer ausreichend vollständigen Gewebezerstörung benutzt werden können. Da die Elektroden 12 am gesamten Umfang der Einrichtung 5 mit entsprechendem Abstand angeordnet werden können, können sie die gewünschte praktisch lückenlose Beaufschlagung des Gewebes an der Mündung 2 und damit dessen Zerstörung bewirken. Gegebenenfalls kann aber über den röhrchenförmigen Anschluss 10 oder eine diesen enthaltende Bedienungsstange auch noch eine geringfügige Drehung relativ zu dem Verschluss 5 durchgeführt werden.

Ferner können bei oder an den einzelnen Elektroden 12 Temperaturfühler installiert sein, um die Hochfrequenzablation auch kontrollieren zu können.

In Figur 2 erkennt man an der Einrichtung 5, die dabei als Metallkäfig gestaltet ist, welcher die Elektroden 12 trägt, eine Dichtung 13, die diese Einrichtung 5 außenseitig umfasst und übergreift, damit die Absaugung zwischen dem Verschluss 6 und dieser Einrichtung 5 effektiv durchgeführt und der Unterdruck dauerhaft erhalten bleiben können. Beispielsweise kann diese Dichtung 13 aus Silikon bestehen.

Die Vorrichtung 1 dient zum Ablatieren oder Koagulieren der Mündung 2 oder des Ausgangs von Lungenvenen 3 im linken Vorhof 4 eines menschlichen Herzens, wodurch ein Vorhofflimmern oder die Neigung dazu beseitigt werden kann. Die Vorrichtung 1 hat dazu eine Einrichtung 5 zum Zerstören des Gewebes an der Mündung 2 und außerdem wenigstens einen in die zu behandelnde Lungenvene 3 von der Mündung 2 aus einführbaren Verschluss 6, der in Gebrauchsstellung den Zufluss von Blut aus der Lunge in den Vorhof 4 und damit zu der Mündung 2 mit Abstand zu dieser Mündung 2 unterbindet. Durch einen an der Vorrichtung 1 anlegbaren, zwischen Einrichtung 5 und Verschluss 6 wirksamen Saugdruck kann der Querschnitt der Lungenvene 3 im Mündungsbereich verkleinert werden, was sie Effektivität bei der Zerstörung des Gewebes an der Mündung 2 verbessert und/oder eine kleiner bemessene Einrichtung 5 erlaubt.

## Patentansprüche

1. Vorrichtung (1) zum Ablatieren oder Koagulieren der Mündung (2) oder des Ausgangs der Lungenvenen (3) im linken Vorhof (4) eines menschlichen Herzens zur Beseitigung eines Vorhofflimmerns, mit einer Einrichtung (5) zum Zerstören des Gewebes am Ausgang der Lungenvenen, wobei zu der Vorrichtung (1) wenigstens ein in die zu behandelnde Lungenvene (3) einführbarer Verschluss (6) gehört, der in Gebrauchsstellung den Zufluss von Blut aus der Lunge in den Vorhof (4) mit Abstand zur Mündung (2) oder zu dem Ausgang der Lungenvene (3) unterbindet, **dadurch gekennzeichnet, dass** die Vorrichtung (1) eine Saugeinrichtung aufweist, deren Einlass in Gebrauchsstellung zwischen dem Verschluss (6) und der Einrichtung (5) zur Gewebezerstörung angeordnet ist.

2. Vorrichtung nach Anspruch 1, **dadurch gekennzeichnet, dass** der Eintritt in die Saugeinrichtung eine gelochte oder durchlöcherte Schlauch- oder Röhrchen- oder Metallhülse ist, die einen schlauch- oder röhrchenförmigen Anschluss (10) hat.

3. Vorrichtung nach Anspruch 1 oder 2, **dadurch gekennzeichnet, dass** der Verschluss (6) als Ballon ausgebildet ist, der durch einen Zuführkatheter (7) vom Vorhof (4) des Herzens in die Lungenvene (3) einführbar und dann aufweitbar ist.

4. Vorrichtung nach einem der Ansprüche 1 bis 3, **dadurch gekennzeichnet, dass** der Verschluss (6) an seiner Peripherie oder Außenseite Verankerungsmittel hat.

5. Vorrichtung nach einem der Ansprüche 1 bis 4, **dadurch gekennzeichnet, dass** als Verankerungsmittel an dem Verschluss (6) oder Ballon ein diesen Verschluss (6) oder Ballon umschließendes Geflecht (8) aus Fäden oder Drähten, beispielsweise aus Nitinoldrähten, vorgesehen ist.

6. Vorrichtung nach einem der Ansprüche 1 bis 5, **dadurch gekennzeichnet, dass** die Saughülse auf einer Zuführstange oder auf einem Zuführschaft für den Verschluss (6) koaxial zu dieser Zuführstange, aber mit radialem Abstand dazu angeordnet ist.

7. Vorrichtung nach einem der Ansprüche 1 bis 6, **dadurch gekennzeichnet, dass** die Einrichtung zur Gewebezerstörung ein mit einem Kältemittel zu füllender Ballon und/oder eine metallische Elektrode ist, die einen ununterbrochenen Umfang zur Beaufschlagung der Mündung der jeweiligen Lungenvene hat.

8. Vorrichtung nach einem der Ansprüche 1 bis 7, **dadurch gekennzeichnet, dass** sie einen Führungsdraht (11) aufweist und zumindest der Verschluss (6) einen den Führungsdraht (11) verschiebbar umfassenden Kanal oder Durchtritt hat.

9. Vorrichtung nach einem der Ansprüche 1 bis 8, **dadurch gekennzeichnet, dass** der Verschluss (6) und die Einrichtung (5) zum Zerstören des Gewebes mittels einer in Gebrauchsstellung gespannten Zugfeder (9), vorzugsweise einer Schraubenfeder, verbunden sind.

10. Vorrichtung nach einem der Ansprüche 1 bis 9, **dadurch gekennzeichnet, dass** die insbesondere als Zugfeder (9) ausgebildete Verbindung zwischen dem Verschluss (6) und der Einrichtung (5) zum Behandeln des Gewebes aus elektrisch leitfähigem Werkstoff oder aus zumindest teilweise blankem Metall besteht und als Elektrode wirkend mit einer Signalaufnahmeeinrichtung verbunden ist.
